# EUROPEAN PATENT APPLICATION

(11) **EP 3 702 448 A1**
(43) Date of publication of application: **02.09.2020**
(21) Application number: 19160315.8
(22) Date of filing: 01.03.2019
(51) Int. Cl.: C12N 5/0793, G01N 33/483, G01N 33/68

(54) **NEURONAL NETWORK AND METHOD FOR MONITORING EXCITATORY AND INHIBITORY BALANCE**

(71) Applicant: NeuroProof GmbH, 18119 Rostock (DE)
(72) Inventor: Schröder, Olaf, 04347 Leipzig (DE); Jügelt, Konstantin, 18119 Rostock (DE)
(74) Representative: Moré, Solveig Helga

(57) **Abstract**

The present invention relates to the field of mental disorders associated with an excitatory and inhibitory imbalance such as schizophrenia, bipolar disorders, Alzheimer's disease, Parkinson's disease, or autism spectrum disorders. In particular, the invention provides a neuronal network comprising excitatory and inhibitory neurons characterized by an excitatory and inhibitory imbalance, and uses thereof. It may be used as a disease model, e.g., for schizophrenia, in particular, in methods of identifying novel antipsychotic drugs. The invention also provides a method for analyzing excitatory and inhibitory balance in neuronal networks comprising excitatory and inhibitory neurons comprising stimulating the excitatory neurons, inhibiting the excitatory neurons, stimulating the inhibitory neurons and/or inhibiting the inhibitory neurons. Analysis of neuronal networks of interest, e.g., from a subject suspected of having a disorder associated with an excitatory and inhibitory imbalance can be compared with this analysis, providing a method for diagnosing a condition associated with an excitatory and inhibitory imbalance in a subject.

## Description

The present invention relates to the field of mental disorders associated with an excitatory and inhibitory imbalance such as schizophrenia, bipolar disorders, Alzheimer's disease, Parkinson's disease, ADS, ADHS, depression or autism spectrum disorders. In particular, the invention provides a neuronal network comprising excitatory and inhibitory neurons characterized by an excitatory and inhibitory imbalance, and uses thereof. It may be used as a disease model, e.g., for schizophrenia, in particular, in methods of identifying novel antipsychotic drugs. The invention also provides a method for analyzing excitatory and inhibitory balance in neuronal networks comprising excitatory and inhibitory neurons comprising stimulating the excitatory neurons, inhibiting the excitatory neurons, stimulating the inhibitory neurons and/or inhibiting the inhibitory neurons. Analysis of neuronal networks of interest, e.g. from a subject suspected of having a disorder associated with an excitatory and inhibitory imbalance can be compared with this analysis, providing a method for diagnosing such a condition.

### 1 Background of the invention

Autism spectrum disorders and Schizophrenia are cognitive disorders with complex genetic architectures but overlapping behavioral phenotypes, which suggests common pathway perturbations. Multiple lines of evidence implicate imbalances in excitatory and inhibitory activity (E/I imbalance) as a shared pathophysiological mechanism (Gao and Penzes 2015).

Schizophrenia is a brain disorder affecting mental functions and behavior for about one percent of the world population. The outcomes are associated with a variable course and range from complete recovery to severe disability. The disease is characterized by hallucinations, delusions, and disorganization, which may lead to dangerous or bizarre behaviors. Together with negative symptoms, such as social withdrawal and diminished emotional engagement, and cognitive impairments, schizophrenia can significantly impair social and occupational functioning.

Neurobiological and environmental factors cause schizophrenia. The disease is a severe chronic and disabling brain disorder. The onset of schizophrenia typically begins in early adulthood, but an individual may be diagnosed at any age. Existing treatment options are dedicated to positive symptoms and not related to the prevention of psychotic relapse in patients with schizophrenia. The development of therapies for schizophrenia needs to include early treatment options for the first episodes of psychosis (Millan, Andrieux et al. 2016). It has been recognized by research that the earlier the first episode of psychosis is diagnosed and treated, the more an effective treatment can be expected. Many of the currently available medications for psychosis show severe side effects.

Schizophrenia is a multifactorial disease in which genetic causes may play a role, as shown in twin studies. However, genome-wide association studies have demonstrated that no single genetic locus alone can yield a high risk of developing schizophrenia.

Other factors represent bacterial and viral infections as a result of infections *in utero,* e.g. with *Neisseria Gonorrhoeae, Herplex Simplex* or other infections. It is estimated that 40% of schizophrenia patients have an increased level of inflammation biomarkers.

Research has been concentrating on a number of schizophrenia-specific disease models and hypotheses.

Research shows that a disease phenotype of schizophrenia can be characterized as a disruption of the excitatory inhibitory (E/I) balance to the excitatory side. The disturbed E /I balance with increased excitatory side is caused by a hypofunction of the NMDA receptors placed on GABAergic cells, which causes a disinhibition of GABAergic cells. This presents a specific type of disturbed E/I balance to the excitatory side.

The hypothesis related to NMDA receptors assuming a hypofunction of the NMDA receptors in schizophrenia (Frohlich and Van Horn 2014) resulted in the development of test procedures based on NMDA antagonists. A test procedure using the NMDA antagonist PCP is published in WO 99/20315 A1. Novel antipsychotics identified on this basis should be able to treat cognitive symptoms, as the NMDA receptor is closely linked to the neuronal plasticity and learning processes.

The dopamine hypothesis assumes that hyperactivity of the dopaminergic cells leads to schizophrenic symptoms. The typical and atypical antipsychotics act as antagonists at the dopamine receptors, with binding to the D2 receptor being the preferred binding site of all antipsychotics (Remington 2015) today.

There are a number of indications that schizophrenia can be caused by a defective neuronal development (Insel 2010). The inflammatory hypothesis assumes that an infection mediates a chronic infection that influences neuronal development (Müller 2018). Infections caused by influenza, herpes viruses or Neisseria gonorrhoeae infection during pregnancy, the latter in the first trimester, increase the likelihood of developing schizophrenia (Babulas, Factor-Litvak et al. 2006 Schaefer, & Brown, 2006). Reuss and co-workers have shown that treatment of neuronal cell cultures or monkeys with anti-*Neisseria gonorrhoeae* antibodies can cause changes in the neuronal development (Reuss, Asif et al. 2016, Almamy, Schwerk et al. 2017 2014).

The Poly (I:C) model by (Piontkewitz, Arad et al. 2012 2012) is known in literature as a model of schizophrenia. With the peptide poly (I:C), a viral infection is nonspecifically modeled. The peptide Poly-inosinic: polycytidylic acid, poly(I:C) is similar to double-stranded RNA and stimulates the immune system. In rats, schizophrenia-like behavior was observed when Poly (I:C) was given.

The Methylazoxymethanol Acetate, short: MAM model is a model in which neuronal development is terminated, so that schizophrenia-like behavior in rats were generated. The model has to be calibrated very accurately to obtain schizophrenia-like behaviors of the rat. Precise treatment of the pregnant rat with MAM is crucial for the reproducibility of the results. For this reason an in vitro MAM model couldn't be established yet successfully. Controlling schizophrenic behavior in vitro would permit to adjust precisely dosage and time of MAM.

iPSC based disease models with patient-derived cell material differentiated into neuronal cells, especially for schizophrenia, have been discussed, but are considered dangerous (Jacobs 2015). Schizophrenia is a multifactorial disease with multigenetic causes. iPSC reprogramming also causes mutations, which could prevent or cause schizophrenia. The differentiating process of iPSC to neurons is very complex and can influence schizophrenic or non-schizophrenic behavior dramatically. Another aspect is that iPSC reprogramming and neuronal differentiation can also over disturb neuronal activity, which then has a more epileptogenic than schizophrenic phenotype .

A tool which would help to control *in vitro* whether the phenotype of such cell cultures is schizophrenic or not, would help to validate and finally to establish relevant disease models.

While the complexity of the matter cannot be satisfactory resolved by *in vivo* techniques, and drug discovery also is preferentially done *in vitro,* most models of schizophrenia and related diseases are focused on animal models, mainly rats and mice (Jones, Watson et al. 2011 2011).

Therefore, there is a need in the art for a predictive *in vitro* disease model, allowing for, e.g., an assay for antipsychotics, which would allow faster and more effective phenotypic screening and more cost-effective identification of substances compared to animal models. Under such conditions, animal testing may potentially also be avoided or reduced. Disadvantages of the prior art are overcome by the present invention.

### 2 Summary of the Invention

Embodiment 1) A neuronal network comprising excitatory and inhibitory neurons *in vitro,* wherein the neuronal network is derived from cells contacted with a disease mimicking agent selected from the group consisting of antibodies to *Neisseria gonorrhoeae,* antibodies to *Herpes simplex,* LPS, a pro-inflammatory cytokine, another pro-inflammatory agent, MAM and Poly (I:C), under conditions suitable for influencing neuronal development, wherein the neuronal network activity is characterized by an excitatory and inhibitory imbalance.
Embodiment 2) A method for analyzing excitatory and inhibitory balance in neuronal networks comprising excitatory and inhibitory neurons, wherein, optionally, the method is an *in vitro* method and the neuronal networks are in cell culture, the method comprising
   a) detecting an electrophysiological activity of a neuronal network under conditions of
      i) stimulation of the excitatory neurons with a first stimulus; wherein a first activity is detected; and/or
      ii) inhibition of the excitatory neurons with a second stimulus; wherein a second activity is detected; and/or
      iii) stimulation of the inhibitory neurons with a third stimulus; wherein a third activity is detected; and/or
      iv) inhibition of the inhibitory neurons with a fourth stimulus; wherein a fourth activity is detected;
      wherein the electrophysiological activity is at least detected under conditions of i) or iv) and under conditions of ii or iii), wherein, preferably, the electrophysiological activity is detected under conditions i), ii), iii) and iv).
Embodiment 3) A method for analyzing excitatory and inhibitory balance in a neuronal network of interest comprising excitatory and inhibitory neurons, wherein, optionally, the method is an *in vitro* method and the neuronal network is in cell culture, said method comprising
   a) detecting a basic electrophysiological activity of said neuronal network;
   b) carrying out the method of embodiment 2 with neuronal networks derived from at least one reference subject, preferably, a healthy reference subject;
      wherein steps a) and b) can be carried out in any order;
   c) comparing said basic electrophysiological activity of step a) with the electrophysiological activities determined in step b).
Embodiment 4) A method for analyzing excitatory and inhibitory balance in a neuronal network of interest comprising excitatory and inhibitory neurons, wherein, optionally, the method is an *in vitro* method and the neuronal network is in cell culture, the method comprising
   a) detecting a basic electrophysiological activity of said neuronal network;
   b) comparing said basic electrophysiological activity with the electrophysiological activities determined by carrying out the method of claim 2 with neuronal networks derived from at least one reference subject, preferably, a healthy reference subject.
Embodiment 5) The method of any of embodiment 3 or 4, further comprising contacting the neuronal network of interest or a neuronal network having the same origin as the neuronal network of interest with a potential antipsychotic agent and detecting the electrophysiological activity of said network under the influence of said potential antipsychotic agent.
   The method of any of embodiment 2-5, wherein the first, second, third and/or fourth stimulus are chemical or biological agents selected from the group comprising a neurotransmitter, an inhibitor of a neurotransmitter, an antibody, a RNA interference approach with shRNA and CRISPR/CAS targeting main excitatory and inhibitory pathways such as GABA and Glutamate receptors.
Embodiment 6)
   wherein the first stimulus is a GABA receptor antagonist selected from the group comprising Pentylentetrazole, Picrotoxin and Bicuculline, and/or
   wherein the second stimulus is a Glutamate receptor agonist selected from the group comprising NMDA (N-Methyl-D-aspartic acid) and AMPA (α-amino-3-hydroxy-5-methyl-4-isoxazolepropionic acid), and/or
   wherein the third stimulus is a NMDA or AMPA receptor antagonist selected from the group comprising Ketamine, MK801 or NBQX, and/or
   wherein the fourth stimulus is a GABA-A or GABA-B receptor agonist selected from the group comprising Diazepam, Clonazepam, Zolpidem or SKF97541.
Embodiment 7) The method of any of embodiment 2-6, wherein the electrophysiological activity is determined by a method selected from the group consisting of spike train activity, calcium imaging and field potential recording, preferably, by spike train analysis.
Embodiment 8) The method of any of embodiment 2-7, wherein said comparing step is carried out with a computer program product suitable for comparing spike train patterns selected from the group of computer program products comprising a spike train learner and a spike train model, wherein the computer program product preferably uses artificial intelligence trained on reference neuronal networks for said comparison. The spike train learner can be trained with parameters computed by spike train feature algorithms or directly with the spike trains with algorithms using pulsed neuronal artificial networks.
Embodiment 9) The method of any of embodiment 2-8, wherein the neuronal network is derived from cells contacted with a disease mimicking agent selected from the group consisting of antibodies to *Neisseria gonorrhoeae,* antibodies to *Herpes simplex,* LPS, a pro-inflammatory cytokine, another pro-inflammatory agent, MAM and Poly (I:C), under conditions suitable for influencing neuronal development, wherein, preferably, the neuronal network is derived from cells contacted with said agent *in vitro.*
Embodiment 10) The neuronal network of embodiment 1 or the method of any of embodiments 2-9, wherein the neuronal network is derived from cells contacted with antibodies to *Neisseria gonorrhoeae* selected from the group comprising polyclonal antibodies, wherein, preferably, the neuronal network is derived from cells contacted with said antibodies *in vitro.*
Embodiment 11) The neuronal network of any of embodiments 1 or 10 or the method of any of embodiments 2-10, wherein the neuronal network is derived from cells of a subject suspected of having a condition or having a condition associated with an excitatory and inhibitory imbalance selected from the group comprising schizophrenia, an autism spectrum disorder, bipolar disorder, Alzheimer's disease, Parkinson's disease, ADS, ADHS and depression.
Embodiment 12) The neuronal network of any of embodiments 1 or 10-11 or the method of any of embodiments 2-11, wherein the neuronal network is derived from cells of a subject selected from the group consisting of neuronal progenitor cells, PSC, preferably, iPSC, and non-human embryonic stem cells.
Embodiment 13) The neuronal network of any of embodiments 1 or 10-12 or the method of any of embodiments 2-12, wherein the neuronal network comprises cells derived from a human, a mouse, a rat, a rabbit, a guinea pig, a dog, a cat, a monkey, an ape or a pig, preferably, a human.
Embodiment 14) The neuronal network of any of embodiments 1 or 10-13 or the method of any of embodiments 2-13, wherein the neuronal network comprises glutaminergic neurons, GABAergic neurons and, preferably, dopaminergic neurons.
Embodiment 15) The neuronal network of any of embodiments 1 or 10-14 or the method of any of embodiments 2-14, wherein the neuronal network is in contact with a multi-electrode array.
Embodiment 16) A method of identifying an antipsychotic agent, comprising carrying out the method of any of embodiments 5 to 15.
Embodiment 17) A method of selecting a drug suitable for treatment of a subject having a condition associated with an excitatory and inhibitory imbalance in a subject, comprising carrying out the method of any of embodiments 5-15 with a neuronal network of interest comprising excitatory and inhibitory neurons from cells derived from cells of the subject, preferably, from iPSC,
   wherein the method comprises contacting said neuronal network with at least one drug, preferably, an antipsychotic agent, and detecting the electrophysiological activity of said network under the influence of said drug,
   wherein a drug is suitable for treatment of the condition if the excitatory and inhibitory imbalance in the neuronal network is ameliorated by said drug.
Embodiment 18) An *in vitro* method for diagnosing a condition associated with an excitatory and inhibitory imbalance in a subject suspected of having a condition associated with an excitatory and inhibitory imbalance selected from the group comprising schizophrenia, autism, bipolar disorder, Alzheimer's disease, Parkinson's disease, ADS, ADHS and depression, comprising
   A) optionally, generating a neuronal network of interest comprising excitatory and inhibitory neurons from cells derived from cells of the subject, preferably, from iPSC,
   B) carrying out the method of any of embodiments 11-15, with the neuronal network of interest derived from cells of the subject in step A), wherein an excitatory and inhibitory imbalance detected in said neuronal network is associated with said condition,
   C) optionally, classifying the excitatory and inhibitory imbalance detected in said neuronal network according to a comparison with the results of an analysis of at least one reference subject having said condition, wherein, preferably, said comparison is carried out by a machine learning algorithm or pattern recognition algorithm,
   D) optionally, further providing the diagnosis to the subject or a medical practitioner responsible for the subject.
Embodiment 19) A stimulus suitable for
   i) stimulation of excitatory neurons, designated the first stimulus; and/or
   ii) inhibition of excitatory neurons, designated the second stimulus; and/or
   iii) stimulation of the inhibitory neurons, designated the third stimulus; and/or
   iv) inhibition of the inhibitory neurons, designated the fourth stimulus;
   wherein the first, second, third and fourth stimulus is a chemical or biological agent selected from the group comprising a neurotransmitter, an inhibitor of a neurotransmitter, an antibody, an RNA interference approach, and CRISPR/CAS, targeting main excitatory and inhibitory pathways such as GABA and Glutamate receptors.
   for use in diagnosing a condition associated with an excitatory and inhibitory imbalance, by analyzing excitatory and inhibitory balance in a neuronal network of interest comprising excitatory and inhibitory neurons derived from cells of a subject suspected of having a condition associated with an excitatory and inhibitory imbalance selected from the group comprising schizophrenia, autism, bipolar disorder, Alzheimer's disease, Parkinson's disease, ADS, ADHS and depression, comprising
   a) detecting a basic electrophysiological activity of said neuronal network of interest *in vivo* using a multi-electrode array in contact with said neuronal network;
   b) detecting an electrophysiological activity of reference neuronal networks under conditions of
      i) stimulation of the excitatory neurons with the first stimulus; wherein a first activity is detected; and/or
      ii) inhibition of the excitatory neurons with the second stimulus; wherein a second activity is detected; and/or
      iii) stimulation of the inhibitory neurons with the third stimulus; wherein a third activity is detected; and/or
      iv) inhibition of the inhibitory neurons with the fourth stimulus; wherein a fourth activity is detected;
      wherein the electrophysiological activity is at least detected under conditions of i) or iv) and under conditions of ii or iii), wherein, preferably, the electrophysiological activity is detected under conditions i), ii), iii) and iv)
      wherein steps a) and b) can be carried out in any order;
   c) comparing said basic electrophysiological activity of step a) with the electrophysiological activities determined in step b).
   d) optionally, classifying the excitatory and inhibitory imbalance detected in said neuronal network of interest according to a comparison with the results of an analysis of at least one reference subject having said condition, wherein, preferably, said comparison is carried out by a machine learning or pattern recognition algorithm,
   e) optionally, further providing the diagnosis to the subject or a medical practitioner responsible for the subject.
Embodiment 20) Use of the neuronal network of any of embodiment 1 or 10-14 for analyzing excitatory and inhibitory balance in a neuronal network, preferably, for identifying an antipsychotic agent.
Embodiment 21) Use of a the method of any of embodiments 2-15 for analyzing excitatory and inhibitory balance in a neuronal network, preferably, for identifying an antipsychotic agent, wherein, optionally, the neuronal network is the neuronal network of any of embodiment 1 or 10-14.

### 3 Figure Legends

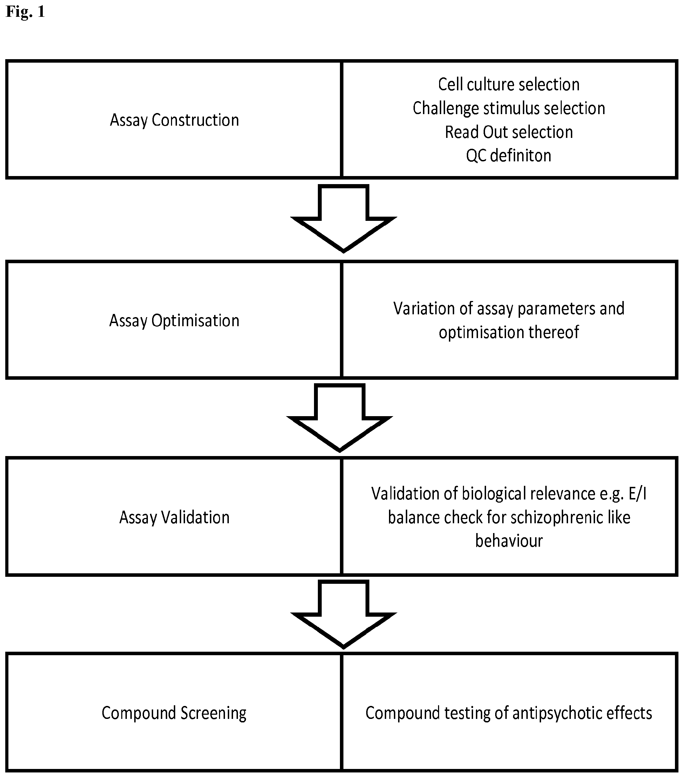

- **Fig. 1**: **Workflow of assay development.** This figure describes the steps in assay development starting from assay construction with a consecutive steps of assay optimization and assay validation, which both can be repeated until a final satisfactory result. The final step is then the compound screening.

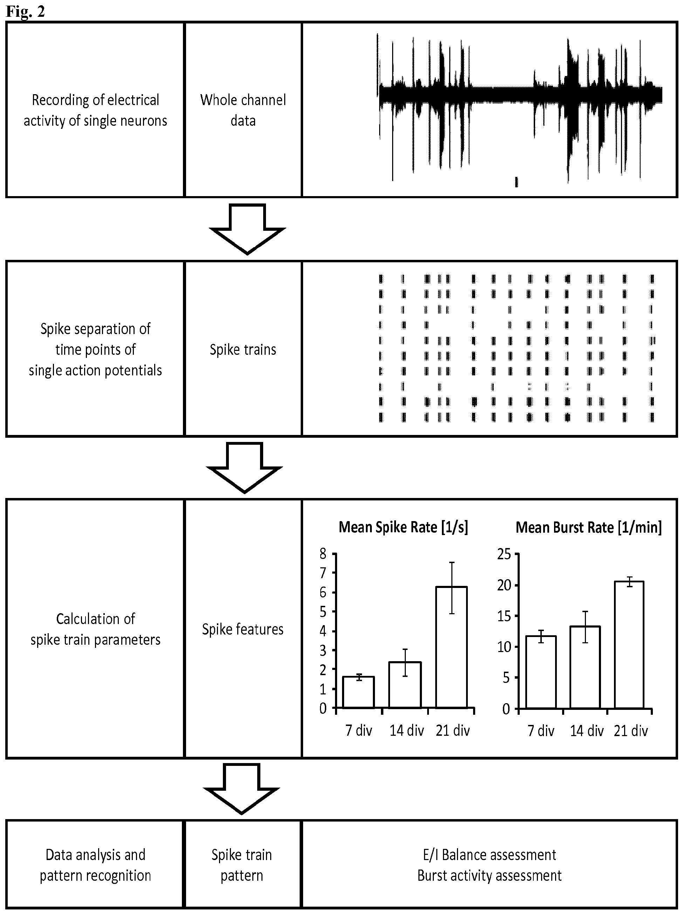

- **Fig. 2**: **Flow diagram for the recording and analysis of electrophysiological activity in neuronal networks by MEA recordings and consecutive steps of data preparation and analysis.** Electrical neuronal activity is recorded with a microelectrode array system. The result are whole channel data containing extra cellular action potentials of single neurons, which can be detected by a peak detection e.g. spike detection algorithm. Time points of action potentials are stored as so called spikes. The whole of spike trains from different channels and neurons is saved as multi spike train or simple spike train. For these spike trains so called spike train features are calculated as the number of spikes per time as spike rate and so forth. The left graph (A) for spike features shows the mean spike rate (Mean +/- SEM [1/s]). for 7 div, 14 div and 21 div (div= days in vitro). The right graph (B) shows the mean burst rate (Mean +/- SEM [1/min]). for 7 div, 14 div and 21 div. With higher data analysis methods as machine learning algorithms, multivariate data analysis complex activity patterns are analyzed.

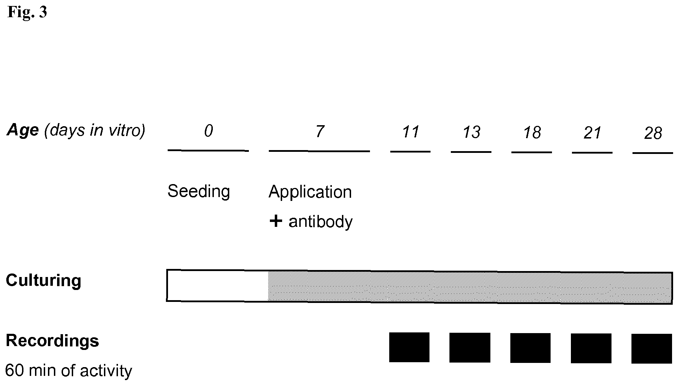

- **Fig. 3**: **Experimental scheme.** Experiments start with cultivation of cells on MEA with age at day 0 in vitro, the seeding. Culturing is performed for 28 days in vitro. Culture medium is changed every 3-4 days. Recording of activity is performed for 60 minutes on day 11, 13, 18, 21 and 28 days in vitro.

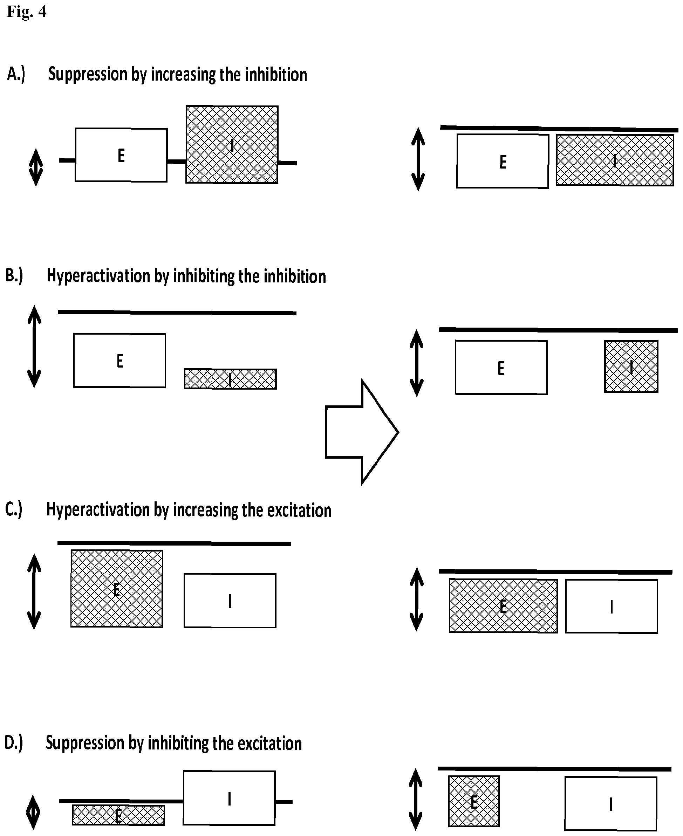

- **Fig. 4**: **Four class model of E/I balance disturbance and homeostasis.** Initial disturbance of E/I balance is presented on the left side. Four types can be distinguished: A) Inhibition by increased inhibition. B) Hyperactivation by reduced inhibition. C) Hyperactivation by increased excitation. D) Hypoactivation by reduced excitation. Activity, indicated by the horizontal line, can be decreased, leading to suppression, or increased, leading to hyperactivation. This disturbance can be caused by an initial miss function of the excitatory or inhibitory system indicated by the letter E or I. For in type A. an increase inhibition leads to a suppression of activity and so forth. However, homeostatic counter regulation. which is indicated with the left to right arrow in the center of the graph, on the right side leads to a balanced level of activation, but since this counter regulation is executed by other homeostatic functions a miss balance of excitation and inhibition is still existing, which is indicated by the areas of E and I.

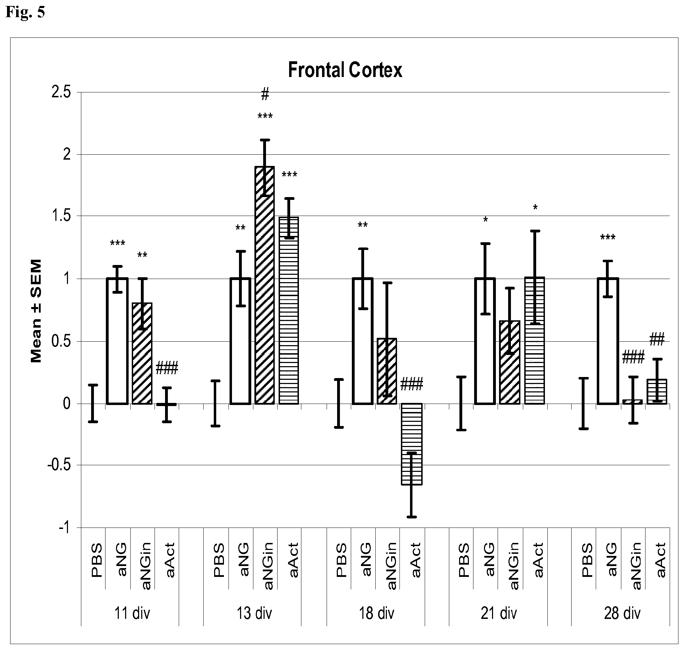

- **Fig. 5**: **Effect score calculation for frontal cortex cultures on DIV 11, 13, 18, 21 and 28.** Frontal cortex cultures were treated with an antibody to Neisseria gonorrhoeae, aNG, and control (PBS), an inactivated antibody to Neisseria gonorrhoeae, aNGin and an antibody to Actinin, aAct. Effect scores were calibrated as a calibration between PBS and aNG for each time point. With this effect score the effect scores for aNGin and aAct were calculated. The best separation was detected at DIV 28.Values are mean ± standard error of mean. * denotes significance against PBS, # against aNG; */**/*** p ≤ 0.5/0.01/0.001.

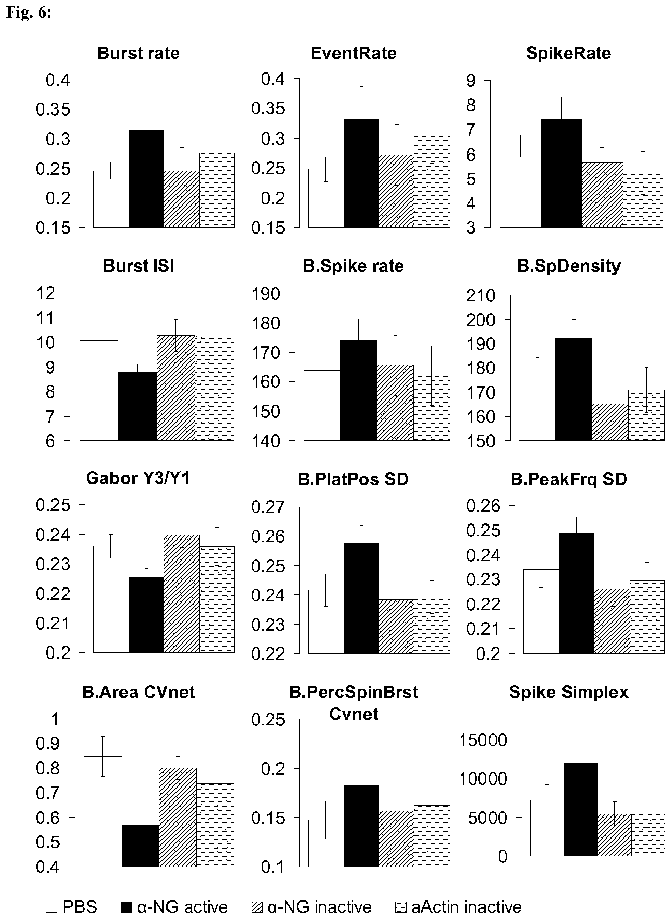

- **Fig. 6**: **Graphs of 12 electrophysiological spike train parameters from frontal cortex cultures at DIV 28.** Frontal cortex cultures were treated with an antibody to Neisseria gonorrhoeae, aNG, and control (PBS), an inactivated antibody to Neisseria gonorrhoeae, aNGin and an antibody to Actinin, aAct. Activity was measured at DIV 28 and spike train parameters were calculated. It is shown that Burst parameter: Burst rate, Burst Spike rate and Burst Spike Density were increased for aNG, which is correlation with an observed increase of theta oscillation in schizophrenic patients. Values are mean ± standard error of mean.

### 4 Description of the Invention

In a first aspect, the present invention provides a model for a disease associated with an excitatory and inhibitory imbalance, preferably, schizophrenia. Said model is preferably used *in vitro,* in the form of a neuronal network in cell culture, or *in vivo.* The invention provides a neuronal network comprising excitatory and inhibitory neurons *in vitro,* wherein the neuronal network is derived from cells contacted with a disease mimicking agent under conditions suitable for influencing neuronal development, wherein the neuronal network activity is characterized by an excitatory and inhibitory imbalance. The disease mimicking agent is selected from the group consisting of antibodies to *Neisseria gonorrhoeae,* antibodies to *Herpes simplex,* LPS, a pro-inflammatory cytokine, another pro-inflammatory agent, MAM and Poly (I:C). Preferably, throughout the invention, antibodies to *Neisseria gonorrhoeae* are the preferred disease mimicking agent.

The inventors have found, that one or more of said agents can be used to induce in a non-human subject or in a neuronal cell culture a state characterized by an excitatory and inhibitory imbalance, e.g., a state similar to schizophrenia. The invention also provides use of a disease mimicking agent, e.g., antibodies to *Neisseria gonorrhoeae* for use in generating a non-human model of a disease associated with an E/I imbalance such as a schizophrenia model, e.g., by contacting a non-human animal with the disease mimicking agent *in utero.* Such a model may be a non-human animal model, e.g., a mouse, a rat, a rabbit, a guinea pig, a dog, a cat, a monkey, an ape or a pig, preferably, a mouse. It may also be a neuronal network in cell culture derived from said non-human animal after the animal has been contacted with the disease mimicking agent *in utero.*

### 4.1 E/I Balance

An "Excitatory and inhibitory balance", or E/I balance describes the balance between excitatory and inhibitory components of the nervous system. The E/I balance is primarily maintained by the balance of glutamatergic and GABAergic neurons, wherein the excitatory system is mainly characterized by glutamatergic neurons and the inhibitory system is mainly characterized by GABAergic neurons (Mullner, Wierenga et al. 2015). If a neuronal system is stimulated, this stimulation is transmitted to further neurons, which then stimulate still further neurons etc. This process however needs to be stopped at some time, because otherwise, the system would use all its resources and would collapse, or an epileptic seizure would be induced. To prevent such a constant hyperactivity or a constant hypoactivity, the neuronal network is tuned utilizing homeostatic control circuits. Homeostasis constantly directs the activity of a neuronal system in a range of tolerance, and tends to maintain an essentially constant level of activation over time (Turrigiano 2008).

The E/I balance may be disturbed when the nervous system has activity in the range of a reasonable bandwidth, but has been primarily adjusted through homeostatic mechanisms. This homeostatic process is described in **Fig. 4****.**

At the level of individual neurons, this balance involves the maintenance of appropriate ratios of excitatory versus inhibitory synaptic inputs. Activation of an excitatory synapse by glutamate depolarizes the cell and increases the likelihood that the cell will fire an action potential while activation of an inhibitory synapse by GABA does the opposite. Thus, the E/I synaptic ratio is critical for keeping the cell's overall firing patterns within a narrow range. However, this ratio is highly asymmetric across development and between neuronal subtypes. Surprisingly, the ratios themselves are tightly regulated with minimal variance even between branches on a single neuron. Neurons typically establish and maintain this long- term internal consistency despite such external variability. In a landmark paper, (Turrigiano, Leslie et al. 1998), discovered chronic activity blockade with tetrodotoxin (TTX) and bicuculline increased and decreased the amplitude of miniature excitatory postsynaptic currents (mEPSC) respectively in cultured neurons, while drug washout restored firing rates to control levels. Thus, in response to outside influences forcibly altering activity, compensatory mechanisms are recruited to restore the initial circuit set point. These responses include modulation of excitatory and inhibitory postsynaptic strength, alterations in presynaptic neurotransmitter release probability, and adjustment of intrinsic membrane excitability, all of which affect and are influenced by neuronal architecture, including dendritic spines and arbors (Gao and Penzes 2015). Multiple levels of homeostatic control exist to ensure the neuron's output is appropriately stable, all of which contribute to E/I balance.

Homeostasis counterregulates the effects of an E/I imbalance (Nelson and Valakh 2015). The primary E/I balance, as a balance of excitatory and inhibitory main drivers, can also be imbalanced even though, due to homeostatic tuning, it has an acitivity in the tolerance range. The counter-regulation of homeostasis is based on other mechanisms than the dysregulation of the E/I balance.

An E/I imbalance is characterized by a deviation from the E/I balance maintained in healthy subjects. Either the excitatory or the inhibitory system may have to be more activated than in physiological situations. For example, a loss of GABA receptors can have the effect that the network is constantly not inhibited in a sufficient manner, which leads to a constant too high excitation, which than has to be counter-regulated by other homeostatic mechanisms **(****Fig. 4****).**

Disorders associated with an E/I imbalance are schizophrenia, autism spectrum disorders (Mok, Nadasdy et al.) such as autism, Alzheimer's disease, Parkinson's disease, bipolar disorder, Alzheimer's disease, ADS (attention deficit syndrome), ADHS (Attention deficit and hyperactivity syndrome) and depression.

For example, in schizophrenia (Kehrer, Maziashvili et al.) or autism, there is a predominance of excitatory activity. For autism, a dysregulation of gabaergic parvalbumin positive neurons has been suggested (Gogolla, Leblanc et al.).

In Alzheimer's disease, in particular, in early phases of Alzheimer's disease, a dysregulation of the GABAergic system has been found, leading to a reduced inhibition by the GABAergic system. This leads to an overall predominance of excitation (Busche and Konnerth 2016)
Examples of dysregulations of the E/I balance are summarized in Table 1.

**Table 1: Examples of dysregulations of the E/I balance**

| E/I Balance type of dysregulation | Indication | Homeostatic counter-regulation | Remarks | Reference |
|---|---|---|---|---|
| Hyperexcitation by reduced inhibition | Alzheimer | | | (Busche and Konnerth 2016) |
| | Autism | | | (Gogolla, Leblanc et al. 2009) |
| | Schizophrenia | | NMDA hypothesis as hypofunction of NMDA receptors on gabaergic neurons. | |
| Hyperexcitation by increased excitation | Acute brain damage, stroke | | NMDA excitotoxicity | |
| Deactivation by inhibition of excitation | | | | |
| Deactivation by increased inhibition | ALS | Increased glutamate level | No epilepsy as comorbidity | (Tuk 2016) |

### 4.2 The Assay Development Process

Assay developmental process is divided in several steps as shown in **Fig. 1****.** Optimal conditions will be defined and the assay will be validated in accordance with prior experiences. Validation of the assay is the critical step in assay development. This assay is constructed based on clinical evidence that infection with Neisseria gonorrhoeae in early pregnancy can cause schizophrenia. It is necessary to demonstrate that the assay mimics symptoms observed in the clinic or at least in animal models of schizophrenia. This invention proposes tools which improve the validation of such an assay, so that the likelihood to have a schizophrenia like in vitro model is increased.

### 4.3 Detection of E/I Imbalance

In the art, E/I imbalances have been determined by imaging techniques, e.g., proton magnetic resonance tomography, (Ford, Nibbs et al. 2017 2017). The GAD-67 Transporter can, for example, be stained as a marker for GABAergic cells. Prange, 2004 quantitatively determine synaptic proteins such as PSD 95, which is specific for excitatory synapses, and neuroligin 1, specific for any synapses, to determine the excitatory inhibitory ratio. However, an exact quantitative analysis by these means is very complicated and can only be carried out with dead tissue, but then not in a functional manner.

Functional methods for determining E/I balance are not known in the art, but they would be useful e.g., as diagnostic methods. Further, an analysis of E/I balance would be important for validation of in vitro models, as, by these means, better and more meaningful in vitro models can be developed.

A particular difficulty in analysis of a disturbed E/I balance is that homeostatic mechanisms regulate the neuronal system at a certain range of the neuronal basic activity, even if the E/I balance is disturbed **(****Fig. 4****).** That means that even if the E/I balance is disturbed, e.g., towards the excitatory side, the spike rate does not necessarily need to be increased. If there is no increase, the network has been counter regulated by a compensatory homeostatic mechanism. However, this needs extra resources and can typically not be maintained for long.

### 4.4 Neuronal In vitro Cultures

A "neuronal network" is characterized by a plurality of neurons which are functionally linked, e.g., by synapses. In the context of the invention, a neuronal network comprises at least 1000, preferably, 10.000-1.000.000 neurons. Neuronal networks of the invention comprise both excitatory and inhibitory neurons. The neuronal network of the invention comprises at least glutaminergic neurons and GABAergic neurons. Excitatory neurons have synapses with vesicles comprising the excitatory neurotransmitter glutamate, i.e., they are designated glutaminergic neurons. Inhibitory neurons have synapses with vesicles comprising the inhibitory neurotransmitter GABA, and they are designated GABAergic neurons. Preferably, the neuronal network of the invention also comprises dopaminergic neurons.

A neuronal network may be a neuronal network in cell culture, which preferably, is cultured in contact with a multi-electrode array in order to allow for an advantageous method of analysis. Neuronal networks may be obtained after a differentiation process from primary cultures, e.g., from mice, rats or other species, from neuronal progenitor cells or pluripotent stem cells, e.g., induced pluripotent stem cells, preferably, human iPSC-induced pluripotent stem cells. Direct differentiation of somatic cells into neuronal cells is an alternative. Neuronal networks may also be derived from non-human embryonic stem cells.

The neuronal network may also be *in vivo,* e.g., in a subject suspected of having a disorder associated with an E/I imbalance, a subject diagnosed as having a disorder associated with an E/I imbalance or a healthy reference subject diagnosed as not having a disorder associated with an E/I imbalance.

Neuronal networks may be co-cultures of neuronal cell cultures, in particular, mixtures of different neuronal cell types (e.g., comprising frontal cortex and midbrain neurons or frontal cortex and hippocampal neurons or co-cultures of neurons and glia cells as astrocytes or and microglia. Glia cells are important players in homeostatic processes, so a co-culture with glia cells is preferable. Co-cultures can have different properties than cultures of single neuronal cell types. Co-cultures can be prepared by manipulation, e.g., mixture of neuronal progenitor cells form different origins or types or by organic evolution, e.g., by addition of different differentiation factors and/or culture in a manner leading to differentiation of different cell types. The application shows that neuronal cell cultures characterized by an excitatory and inhibitory imbalance can be prepared with high reliability, e.g., with frontal cortex neuronal cells.

### 4.5 NG challenge

The contact of the disease mimicking agent with the neuronal network can take place in cell culture, which is preferred to avoid animal experiments. If the cell culture comprises cells capable of producing pro-inflammatory cytokines, (e.g., TNF-alpha, IFN-gamma, IL-1, IL-6), e.g., microglia cells, agents which modulate inflammation can be used, as they affect neuronal development. Antibodies to *Neisseria gonorrhoeae,* or antibodies to *Herpes simplex* are to be used, preferably, antibodies to *Neisseria gonorrhoeae,* can influence neuronal development. For example, polyclonal antibodies to *Neisseria gonorrhoeae* may be used. Alternatively, antibodies, e.g., monoclonal antibodies directed to specific components of *N. gonorrhoeae* and crossreacting with human proteins may be employed, e.g., antibodies to SNAP-23, antibodies to the SNARE complex, anti-HSP60 antibodies, anti-synaptogamin antibodies and/or anti-chmpla or 5 antibodies as well as anti-syntaxin antibodies or other antibodies described as associated with development of schizophrenia, e.g., in (Almamy, Schwerk et al. 2017) (Reuss 2014) and (Reuss, Asif et al. 2016). Preferably, the neuronal network is derived from cells contacted with said antibodies, e.g., said polyclonal antibodies, *in vitro.*

A combination of two or more, three or more or four or more disease mimicking agents may also be used, e.g., a combination of antibody to *N. gonorrhoeae* and poly(I:C).

Culturing of the neuronal network in cell culture comprises the selection of appropriate cells and procedures for culturing the cells contained in the MEA. There are numerous protocols for cell cultures and types of cells available. For example, neuronal network cultures may be prepared and cultured as taught by (Bader, Steder et al. 2017), (Feng, Bader et al. 2018) for frontal cortex neurons or (Weinert, Selvakumar et al. 2015). Cell cultures can be composed of a singular type of cells, such as frontal cortex neurons, or composed of different cell types from different regions of the brain (the frontal cortex, hippocampus, or other regions). Normally, the neuronal networks comprise cells from one species only, but they may also comprise cells from different species, such as mouse, rat or human. Neuronal networks may be cultivated as 3d organoids.

The culturing of cells can last several days up to several weeks. The time span is dependent on the type and origin of the cells. After a defined or predefined number of days or hours, the cells are incubated with a disease mimicking agent, such as antibodies from *Neisseria gonorrhoeae,* preferred is as early as possible after cultivation.

During cell culture, e.g., during differentiation to neuronal cells, during and after formation of functional neuronal networks, the electrophysiological activity of the cells may be analyzed by MEA recording.

In cell culture, conditions suitable for influencing neural development further require contact during the differentiation of neuronal cells. For example, after differentiation of induced pluripotent stem cells to neuronal cells, the contact may take place at a state of neuronal differentiation, when cell do not show Nestin positive cells anymore. The best day of plating on microelectrode arrays and addition of disease stimulating factors as Neisseria gonorrhoeae antibodies has to be analyzed with the proposed method of this patent. During differentiation of embryonic stem cells, e.g., non-human embryonic stem cells, to neurons, contact may take place from embryonic day 12 to 18.

For example, neuronal progenitor cells may be isolated from mice at day 10-18, preferably, day 15-16 of the embryonic stage, e.g., from the frontal cortex, and cultured on MEA. After 2 to 6 days, preferably, 3-4 days in culture, the cells may treated with an antibody to Neisseria gonorrhoeae, e.g., a polyclonal antibody. After about 20-36 days, e.g., 24-28 days in cell culture neuronal networks derived from cells contacted with the antibody to Neisseria gonorrhoeae show a distinctly different electrophysiological activity. It could be demonstrated that these cultures also exhibit a disturbed E/I balance, with an excitatory predominance as in patients with schizophrenia.

Alternatively, in a non-human subject, the contact may take place *by injection,* preferably, during the first trimester of pregnancy, e.g., through an infection of the maternal animal with the disease mimicking agent, in particular, infection with *Neisseria gonorrhoeae,* or *Herpes simplex,* or through administration of the agent to the maternal animal, e.g., i.v. administration or intravaginal infection.

To prepare an *in vivo* neuronal network characterized by an excitatory and inhibitory imbalance, the *in vivo* development after this contact may be continued in a normal manner. Alternatively, to prepare an *in vitro* neural network, neuronal progenitor cells may also be isolated after said contact to generate a neural network of the invention in cell culture.

### 4.6 Microelectrode arrays and E/I Balance

In a preferred embodiment, the neuronal network of the invention is in contact with a multi-electrode array, MEA, which allows for measurement of electrophysiological activity of neurons of the neuronal network. In one embodiment, the invention provides a cell culture comprising a neuronal network of the invention and a multi-electrode array. A multi-electrode array (also designated microelectrode array) is a device that comprises multiple contacts, e.g., plates or shanks through which neural signals can be obtained and, optionally, also delivered, essentially serving as neural interfaces that connect neurons to electronic circuitry. MEAs for cell cultures comprise electrodes for real-time recording of action potentials of neurons. There are two classes of MEAs: implantable MEAs, used in vivo, and non-implantable MEAs, used in vitro. MEA for in vitro use may comprise wells for cell culturing and electrodes for measuring signals. The microelectrode technology enables the extracellular measurement of signals of electro physiologically active cell cultures through passive electrodes via label-free permanent contacts. MEA offer the parallel measurement of several signals with a given spatial distribution so that the activity of several cells and cell types in a cell culture network can be investigated. Microelectrode arrays are commercially available in different formats from a number of vendors, such as Axion Biosystems, Atlanta, GA.

MEA multiwell plates from Axion Biosystems Inc. have, e.g., 48 wells and, at the bottom, a central MEA with 16 passive measuring electrodes. These plates can be used with the measurement system Maestro of Axion Biosystems Inc., which simultaneously, separated by channels, enhances the signals derived from the microelectrodes, filters and digitalizes them. The cultures are maintained at 37°C and held in humid air 10% CO₂ to regulate pH. The raw data can be used to detect action potentials or spikes in real time, e.g., using the software AxIS. These can be saved together with time markers.

The sequence of time markers, so called "spike trains", may be the basis for the later multiparametric spiketrain analysis. The analysis software NPWaveX (NeuroProof GmbH, Rostock), which may be employed, calculates 204 statistic parameters comprising the general activity, the structure of bursts, network synchronisation and the variability over time.

Any array system in any format and embodiment can be used for the proposed assay, as long it is suitable for cell culturing and MEA recording.

The neuronal network of the invention is characterized by an excitatory and inhibitory imbalance. Such an imbalance is preferably determined according to a method of the invention as described herein, but it may also be determined according to methods known in the art, e.g., as described above.

The invention also provides a method of preparing a neuronal network comprising excitatory and inhibitory neurons *in vitro,* wherein the method comprises contacting neuronal progenitor cells with a disease mimicking agent selected from the group consisting of antibodies to *Neisseria gonorrhoeae,* antibodies to *Herpes simplex,* LPS, a pro-inflammatory cytokine, another pro-inflammatory agent, MAM and Poly (I:C), preferably, with antibodies to *Neisseria gonorrhoeae,* under conditions suitable for influencing neuronal development,
generating a neuronal network from said cells,
characterizing the excitatory and inhibitory imbalance, e.g., in comparison to a reference neuronal network derived from cells which have not been contacted with a disease mimicking agent, and
maintaining those neuronal networks which have an excitatory and inhibitory imbalance. Neuronal networks which do not have an excitatory and inhibitory imbalance are discarded or not used. Preferably, the excitatory and inhibitory imbalance is characterized after div 22, e.g., at div 24-35 or 28-32.

Neuronal networks shown to have an excitatory and inhibitory imbalance, as described herein, may be used, e.g., to identify potential drugs suitable for treatment of a disease associated with an E/I imbalance, in particular, to identify a novel antipsychotic. The invention also provides the use of the neuronal network of the invention for analyzing excitatory and inhibitory balance in a neuronal network, preferably, for identifying an antipsychotic agent.

The invention provides a method for analyzing excitatory and inhibitory balance in neuronal networks comprising excitatory and inhibitory neurons, wherein, optionally, the method is an *in vitro* method and the neuronal networks are in cell culture, the method comprising
a) detecting an electrophysiological activity of a neuronal network under conditions of
   i) stimulation of the excitatory neurons with a first stimulus; wherein a first activity is detected; and/or
   ii) inhibition of the excitatory neurons with a second stimulus; wherein a second activity is detected; and/or
   iii) stimulation of the inhibitory neurons with a third stimulus; wherein a third activity is detected; and/or
   iv) inhibition of the inhibitory neurons with a fourth stimulus; wherein a fourth activity is detected;
   wherein the electrophysiological activity is at least detected under conditions of i) or iv) and under conditions of ii or iii). Preferably, throughout the invention, the electrophysiological activity is detected under conditions i), ii), iii) and iv).

Said method is preferably carried out to provide at least one reference for analysis of the E/I balance of a neuronal network of interest. It is therefore also designated method for analyzing excitatory and inhibitory balance in neuronal reference networks. To this end, the method is preferably carried out with a reference network, which are, e.g., derived from a healthy subject, i.e., a subject diagnosed not to have a disorder associated with an E/I imbalance. Alternatively,, it may be carried out in the context of the disease model generated by contact with a disease mimicking agent, as described herein, or from cells of a subject not contacted with a disease mimicking agent.

It is also possible that the reference subject is a subject diagnosed to have a disorder associated with an E/I imbalance, or, preferably, in the context of the disease model generated by contact with a disease mimicking agent (e.g., antibodies to *N. gonorrhoeae*), as described herein, or from cells of a subject contacted with a disease mimicking agent and found to have an E/I imbalance. If the analysis comprises analysis of neuronal cell cultures from a subject diagnosed to have a disorder associated with an E/I imbalance (e.g., schizophrenia), i.e., a reference subject having an E/I imbalance, the analysis preferably further comprises analysis of neuronal cell cultures from a healthy subject diagnosed not to have a disorder associated with an E/I imbalance.

Preferably, in order to provide maximal values for excitation and/or inhibition, the stimulation of i), ii), iii) and/or iv) are carried out under conditions leading to maximum inhibition or excitation, respectively.

In the context of the method of the invention, the first, second, third and/or fourth stimulus may be a chemical or biological agent, e.g., a neurotransmitter, an inhibitor of a neurotransmitter, an antibody, or an RNA interference approach. Alternatively, the analysis may be carried out with a CRISPR/CAS geneticaly engineered culture, as described above....

The first stimulus may be a GABA receptor antagonist selected from the group comprising Pentylentetrazole, Picrotoxin and Bicuculline.
The second stimulus may be a Glutamate receptor agonist from the group comprising NMDA (N-Methyl-D-aspartic acid) and AMPA (α-amino-3-hydroxy-5-methyl-4-isoxazolepropionic acid). The third stimulus may be a NMDA or AMPA receptor antagonist from the group comprising Ketamine, MK801 or NBQX.
The fourth stimulus may be a GABA-A or GABA-B receptor agonist from the group comprising Diazepam, Clonazepam, Zolpidem or SKF97541. The classification may be still enhanced if several stimuli from each group are used.

The neuronal networks in which the first, second, third and fourth activity are determined are neuronal networks comparable to each other. For example, they may be derived from a neuronal progenitor cell population which has been identically treated for all neuronal networks identified. They may also be derived from the same subject.

If neuronal networks are highly stimulated or inhibited, the assessment of the E/I balance is easy. It is more difficult if the networks have been regulated to their normal level of excitation by homeostatic mechanisms. As explained, this can also be the case if the E/I balance is disturbed, in particular, in chronic conditions. However, surprisingly, an assessment of the E/I balance is still possible based on the inventor's surprising finding that the spike trains under conditions of E/I imbalance with excitatory predominance and inhibitory predominance strongly differ. Differentiation is possible, e.g., by pattern recognition software.

The method of the invention allows for calibration, wherein e.g., based on artificial intelligence, a pattern recognition algorithm learns to differentiate excitatory from inhibitory patterns of electrophysiological activity.

The method of the invention also provides a method for analyzing excitatory and inhibitory balance in a neuronal network of interest comprising excitatory and inhibitory neurons, wherein, optionally, the method is an *in vitro* method and the neuronal network is in cell culture, said method comprising
a) detecting a basic electrophysiological activity of said neuronal network;
b) carrying out the method for analyzing excitatory and inhibitory balance in neuronal reference networks with neuronal networks derived from at least one reference subject, preferably, a healthy reference subject;
   wherein steps a) and b) can be carried out in any order;
c) comparing said basic electrophysiological activity of step a) with the electrophysiological activities determined in step b).

A basic electrophysiological activity is determined without stimulation of said network, e.g., without addition of chemical or biological agent such as a neurotransmitter, an inhibitor of a neurotransmitter, an antibody, inhibitory RNA, CRISPR/CAS or an electrical stimulus.

The method of the invention also provides a method for analyzing excitatory and inhibitory balance in a neuronal network of interest comprising excitatory and inhibitory neurons, wherein, optionally, the method is an *in vitro* method and the neuronal network is in cell culture, the method comprising
a) detecting a basic electrophysiological activity of said neuronal network;
b) comparing said basic electrophysiological activity with the electrophysiological activities determined by carrying out the method for analyzing excitatory and inhibitory balance in neuronal reference networks with neuronal networks derived from at least one reference subject, preferably, a healthy reference subject.

To this end, the electrophysiological activities determined by carrying out the method for analyzing excitatory and inhibitory balance in neuronal reference networks with neuronal networks derived from at least one reference subject, preferably, a healthy reference subject may be stored in a database, e.g., on a computer readable storage medium. Preferably, said database comprises electrophysiological activities derived from a plurality of healthy and /or ill reference subjects. Optionally, the database further neuronal networks have been derived by different methods in combination with said methods, to enable comparison of the neuronal network of interest with neuronal reference networks derived under conditions as similar as possible (except for contact with the disease mimicking agent, if applicable, or for selection of the healthy or potentially ill subject from which they are derived).

The neuronal network of interest which is to be analyzed in the methods of the invention can be derived from a human or animal subject, e.g., a mouse, rat, dog or rabbit. The reference subject is from the same species, and the network of interest and the reference network have the same origin. The neuronal network of interest may be derived from a healthy subject, or it may be from a patient showing symptoms, e.g., of a disorder associated with an excitatory/inhibitory imbalance, e.g. schizophrenia. It may also be from a subject having a risk of developing such as disorder, e.g., due to genetic or any other predisposing factors. The method of the invention may in this context be used for diagnosis. It may also be from a subject known to have such a disorder, e.g., wherein the method is carried out to determine treatment options.

The method of the invention for analyzing excitatory and inhibitory balance in a neuronal network of interest comprising excitatory and inhibitory neurons, may further comprise contacting the neuronal network of interest or a neuronal network having the same origin as the neuronal network of interest with a potential antipsychotic agent and detecting the electrophysiological activity of said network under the influence of said potential antipsychotic agent. Having the same origin in this context means that the neuronal network has been derived under conditions as similar as possible. For example, the neuronal networks compared should be derived from the same species. They should also be derived from the same cell population, e.g., neuronal progenitor cells, iPSC etc., preferably, prepared at the same date of development under the same conditions.

In the context of any method of the invention, the electrophysiological activity may be determined by a method selected from the group consisting of spike train activity, calcium imaging and field potential recording. Preferably, the electrophysiological activity is determined by spike train analysis (Fig. 2). A spike train analysis determines the sequential action potentials occurring in the neuronal network by recordings from a multi-electrode array (Parenti, Turnaturi et al.) and a spike detection algorithm for detecting time points of action potentials, e.g. spikes, from these recordings. In a sequential step from these spike trains, spike train parameters also called spike train features are calculated, like the spike rate as numbers of spikes per time and other parameters (Bader, Steder et al. 2017). Functional assays are preferentially based on MEA recording techniques, because such assays operate in real time and allow the easy development of new categories of antipsychotics. Under such conditions, a phenotypic *in vitro* model based on diseased and health states allows the implementation of superior drug development steps. *In vitro* and under the view and control of microelectrode arrays, induced disease states offer the introduction of standardized assays and therefore improved results in the identification of improved compounds.

The inventors are making use of the fact that the electrophysiological activity of the neuronal networks of the invention can be advantageously evaluated by the analysis of spike trains, theta oscillation and/or bursts, preferably, a combination thereof. Preferably, the comparison of the electrophysiological activity under different conditions is carried out with a computer program product. Said program may be suitable for comparing spike train patterns, e.g., a computer program product comprising a spike train learner and/or a spike train model. The computer program product preferably uses artificial intelligence trained on reference neuronal networks for said comparison.

An action potential occurs when the membrane potential of a specific axon location rapidly rises and falls. This depolarisation then causes adjacent locations to similarly depolarise. In neurons, action potentials play a central role in cell-to-cell communication by providing for - or, with regard to saltatory conduction, assisting the propagation of signals along the neuron's axon towards synaptic boutons situated at the ends of an axon. These signals can then connect with other neurons at synapses, or to motor cells or glands. Action potentials in neurons are also known as nerve impulses or spikes. The temporal sequence of action potentials generated by a neuron is called "spike train".

Theta Oscillations are oscillations in the human EEG in the range of 4-8 Hertz. Comparable oscillations also exist in non-human animals, e.g., in mice, or can be measured from brain slice cultures or dissociated cultures in vitro. An increase in theta oscillations occurs in acutely schizophrenic patients. Theta oscillations correlate with bursting *in vivo* (Hölscher, Anwyl et al. The Journal of Neuroscience The Journal of Neuroscience).

Bursting, or burst firing, is a general phenomenon of the activation patterns of neurons in the central nervous system and spinal cord where periods of rapid action potential spiking are followed by G0 phase quiescent periods. Bursting is thought to be important in the operation of robust central pattern generators, the transmission of neural codes. Associations of abnormal bursting patterns with neuropathologies such as epilepsy or schizophrenia have been found.

### 4.7 Analysis of E/I balance by software

Preferably, the analysis of electrophysiological activity of the invention involves a computer system detecting action potentials ("spikes") from cells in real-time. The raw data streams may store the data together with the time stamps. The sequence of recorded timestamps, the spike train, preferably is the basis for the later multiparametric spike train analysis.

The analysis software preferably mostly analyses statistical parameters that characterize the general activity, the structure of bursts, the synchronization in the network and the variability over time.

By means of the computer program, preferably, using artificial intelligence, a classification value designating the E/I balance of each neuronal network may be determined. If the neuronal network is derived from a subject such as a subject potentially having a disorder associated with an E/I imbalance, the classification value for said subject can thus be determined. Preferably, the classification value is a discrete parameter.To enable an easier comparison multiple measurements and their classification values can be added. It has to be mentioned that one-dimensional parameters allow a relation like "A is greater than B" as a linear order relation.

By means of the computer program, preferably, using artificial intelligence, an effect score, another (preferably one-dimensional) parameter can be calculated from the electrophysiological activity, e.g., from spike train features, of a neuronal network in the absence and presence of an agent of interest (e.g., a potential antipsychotic drug). The effect score reflects the therapeutic effect as precise as possible. The effect score of different potential antipsychotic agents can be compared, e.g., to find the most suitable antipsychotic agent for treatment of a particular subject, or for identifying a novel antipsychotic agent.

However, for both classification value and effect score, the comparison can also be on a multidimensional level. Suitable computer programs are e.g., available from NeuroProof GmbH, Rostock, Germany, e.g., NPWaveX and classification tool PatternExpert.

In one embodiment of the method of the invention, the neuronal network (e.g., the neuronal network of interest) may be derived from cells contacted with a disease mimicking agent selected from the group consisting of antibodies to *Neisseria gonorrhoeae,* antibodies to *Herpes simplex,* LPS, a pro-inflammatory cytokine, another pro-inflammatory agent, MAM and Poly (I:C), under conditions suitable for influencing neuronal development. Preferably, the neuronal network is derived from cells contacted with said agent *in vitro.* Such neuronal networks are further characterized herein, e.g., above or in the experimental part.

In the neuronal network of the invention and the method of the invention, the neuronal network may in one embodiment be derived from cells of a subject suspected of having a condition or having a condition associated with an excitatory and inhibitory imbalance, e.g., schizophrenia, autism, bipolar disorder, Alzheimer's disease, Parkinson's disease, ADS, ADHS and depression. Preferably, throughout the invention the condition (also designated disorder or disease) is schizophrenia. In the context of diagnosis, the subject is suspected of having a condition or having a condition associated with an excitatory and inhibitory imbalance.

In the neuronal network of the invention and the method of the invention, the neuronal network may be derived from cells of a subject selected from the group consisting of neuronal progenitor cells, PSC, preferably, iPSC, and non-human embryonic stem cells. Preferably, the neuronal network is derived from iPSC. To this end, iPSC (e.g., from a human subject or a mouse, preferably, from a human subject) can be prepared, e.g., according to methods known in the art (Shi, Inoue et al. 2017), and neuronal networks can be prepared e.g., according to methods also known in the art (Brennand, Simone et al. 2011).

The neuronal network comprises may comprise cells derived from a human, a mouse, a rat, a rabbit, a guinea pig, a dog, a cat, a monkey, an ape or a pig, preferably, a human.

As discussed above, the neuronal network of the invention or used in the context of the invention comprises glutaminergic neurons, GABAergic neurons and, preferably, dopaminergic neurons. It may further comprise microglial cells, astrocytes and other glia cells.

The neuronal network of the invention may be derived from neuronal progenitors derived from at least one brain region, e.g. frontal cortex, hippocampus, midbrain, hypothalamus, amygdala, preferably, frontal cortex.

Throughout the invention, the neuronal network preferably is in contact with a multi-electrode array.

In one embodiment, the present invention provides a method of identifying an antipsychotic agent, comprising carrying out the method for analyzing excitatory and inhibitory balance in a neuronal network of interest comprising excitatory and inhibitory neurons, comprising contacting the neuronal network of interest or a neuronal network having the same origin as the neuronal network of interest with a potential antipsychotic agent and detecting the electrophysiological activity of said network under the influence of said potential antipsychotic agent. Accordingly, potential antipsychotic agents can be screened by the method or assay of the invention for their capabilities in balancing an imbalances neuronal network.

In comparison to drug screening assays for disorders associated with an E/I imbalance known in the art, the method of the invention has the advantage that it allows for identification of a new class of antipsychotics that may have not only symptomatic but also disease-modifying effects. The novel type of assay is a phenotypic assay. Phenotypic assays are expected as being highly effective for drug testing (Swinney 2013).

The assay of the invention can be validated symptomatically, i.e., it can be tested that characteristics of the electrophysiological activity analyzed directly correlate with clinical symptoms. Furthermore, the assay can be predictively validated, i.e., known substances are analyzed with the assay and correctly assessed with regard to their therapeutic effect.

The inventors have generally shown that the screening assay disclosed herein, e.g., on the basis of a neuronal network treated with antibodies to *Neisseria gonorrhoeae* as disease mimicking agent is valid. In particular, in a substantial percentage of neuronal networks obtained in this way, about 50%, an E/I imbalance was found. The screening assays were also found predictive for effects of known antipsychotic agents. After the addition of antibodies to *Neisseria gonorrhoeae* as a disease mimicking agent, the neuronal networks show a distinctly different activity pattern than untreated cultures. Interestingly, these activity patterns are similar to those patterns of activity in which the neuronal cell cultures were treated with an NMDA antagonist. NMDA antagonists are used in the art to generate models for schizophrenia.

Another validation method utilizes the burst analysis as symptomatic validation. Schizophrenia is characterized by an increase in theta oscillations in patients (Uhlhaas 2013). Theta oscillations are represented *in vitro* as population bursts (Hölscher, Anwyl et al. The Journal of Neuroscience). A comparison of untreated neuronal networks with neuronal network treated with antibodies to *Neisseria gonnorrhoeae* as disease mimicking agent revealed a corresponding change in population bursts characteristic for schizophrenia.

Preferably, neuronal networks of the invention generated by contact with a disease mimicking agent are validated, e.g., analyzed for their E/I balance before they are used in the method of identifying an antipsychotic agent (i.e., the screening assay) of the invention, and neuronal networks having an E/I imbalance are preferably used for the screening assay.

Alternatively, or preferably, additionally, the suitability of the neuronal networks can be validated by burst analysis and/or by analysis of theta oscillations.

The screening assay of the invention may be carried out in a two-step process. In a first step, potentially active agents, optionally, in a plurality of different concentrations, are analysed on neuronal networks derived from healthy subjects, or from neuronal networks not contacted with a disease mimicking agent. Agents having an effect on the E/I balance, and relevant concentrations are thus determined. For the second step of screening, those substances having an effect on the E/I balance which is similar to the effect of reference substances, e.g., known antipsychotic agents, are preferred.

In the second step, the selected agents are tested on neuronal networks of the invention having an E/I imbalance, preferably, neuronal networks comprising excitatory and inhibitory neurons *in vitro,* wherein the neuronal networks are derived from cells contacted with a disease mimicking agent under conditions suitable for influencing neuronal development, as described herein. Alternatively, the neuronal networks having an E/I imbalance are derived from a subject diagnosed as having a disease associated with an E/I imbalance, such as schizophrenia. The results of said analysis are compared with results from neuronal networks not having an E/I imbalance, with neuronal networks having an E/I imbalance in the absence of an agent capable of affecting the E/I balance, and/or with neuronal networks having an E/I imbalance in the presence of known antipsychotic agent. By projection into a one-dimensional effect score, a ranking of agents can advantageously be determined.

Similarly, the invention provides a method of selecting a drug suitable for treatment of a subject, preferably, a human subject, having a condition associated with an excitatory and inhibitory imbalance in a subject, comprising carrying out the method for analyzing excitatory and inhibitory balance in a neuronal network of interest comprising excitatory and inhibitory neurons, comprising contacting the neuronal network of interest or a neuronal network having the same origin as the neuronal network of interest with a drug potentially suitable for treatment of said subject, preferably, an antipsychotic agent, and detecting the electrophysiological activity of said network under the influence of said drug with a neuronal network of interest comprising excitatory and inhibitory neurons from cells derived from cells of the subject. Preferably, the neuronal network is derived from iPSC of a human subject.

A drug is suitable for treatment of the condition if the excitatory and inhibitory imbalance in the neuronal network is ameliorated by said drug.

The invention also provides a method of treatment of a condition associated with an excitatory and inhibitory imbalance in a subject (e.g., schizophrenia), comprising
a) carrying out said method of selecting a drug suitable for treatment of a subject; and
b) administering a drug found suitable for treatment of the condition in step a) to said subject.

In another embodiment, the invention provides an *in vitro* method for diagnosing a condition associated with an excitatory and inhibitory imbalance in a subject suspected of having a condition associated with an excitatory and inhibitory imbalance, e.g., as described herein, comprising
A) optionally, generating a neuronal network of interest comprising excitatory and inhibitory neurons from cells derived from cells, preferably, from iPSC, of a subject suspected of having a condition associated with an excitatory and inhibitory imbalance,
B) carrying out the method for analyzing excitatory and inhibitory balance in a neuronal network of interest of the invention, with the neuronal network of interest derived from cells of the subject of step A), wherein an excitatory and inhibitory imbalance detected in said neuronal network is associated with said condition,
C) optionally, classifying the excitatory and inhibitory imbalance detected in said neuronal network is according to a comparison with the results of an analysis of at least one reference subject having said condition, wherein, preferably, said comparison is carried out by a machine learning algorithm or pattern recognition algorithm,
D) optionally, further providing the diagnosis to the subject or a medical practitioner responsible for the subject.

Before step A), the cells, e.g., cells suitable for preparing iPSC, are obtained from the patient. For example a blood sample may be obtained from the patient, and suitable cells may be isolated. The present invention also provides a stimulus suitable for
i) stimulation of excitatory neurons, designated the first stimulus; and/or
ii) inhibition of excitatory neurons, designated the second stimulus; and/or
iii) stimulation of the inhibitory neurons, designated the third stimulus; and/or
iv) inhibition of the inhibitory neurons, designated the fourth stimulus;
wherein the first, second, third and fourth stimulus is a chemical or biological agent selected from the group comprising a neurotransmitter, an inhibitor of a neurotransmitter, an antibody, an RNA interference approach, and CRISPR/CAS, as further exemplified herein,
for use in diagnosing a condition associated with an excitatory and inhibitory imbalance, by analyzing excitatory and inhibitory balance in a neuronal network of interest comprising excitatory and inhibitory neurons derived from cells of a subject suspected of having a condition associated with an excitatory and inhibitory imbalance, e.g., as described herein, comprising
a) detecting a basic electrophysiological activity of said neuronal network of interest *in vivo* using a multi-electrode array in contact with said neuronal network;
b) detecting an electrophysiological activity of reference neuronal networks under conditions of
   i) stimulation of the excitatory neurons with the first stimulus; wherein a first activity is detected; and/or
   ii) inhibition of the excitatory neurons with the second stimulus; wherein a second activity is detected; and/or
   iii) stimulation of the inhibitory neurons with the third stimulus; wherein a third activity is detected; and/or
   iv) inhibition of the inhibitory neurons with the fourth stimulus; wherein a fourth activity is detected;
   wherein the electrophysiological activity is at least detected under conditions of i) or iv) and under conditions of ii or iii), wherein, preferably, the electrophysiological activity is detected under conditions i), ii), iii) and iv)
   wherein steps a) and b) can be carried out in any order;
c) comparing said basic electrophysiological activity of step a) with the electrophysiological activities determined in step b).
d) optionally, classifying the excitatory and inhibitory imbalance detected in said neuronal network of interest according to a comparison with the results of an analysis of at least one reference subject having said condition, wherein, preferably, said comparison is carried out by a machine learning algorithm or pattern recognition algorithm,
e) optionally, further providing the diagnosis to the subject or a medical practitioner responsible for the subject.

The invention also provides a method of treatment of a condition associated with an excitatory and inhibitory imbalance in a subject, comprising
a) carrying out a method of diagnosis of the invention; and
b) administering a drug suitable for treatment of the condition with which the subject has been diagnosed to said subject.

The invention also provides the use of a the methods of the invention described herein for analyzing excitatory and inhibitory balance in a neuronal network, preferably, for identifying an antipsychotic agent. Optionally, the neuronal network is the neuronal network of the invention derived from cells contacted with a disease mimicking agent selected from the group consisting of disease mimicking agent selected from the group consisting of antibodies to *Neisseria gonorrhoeae,* antibodies to *Herpes simplex,* LPS, a pro-inflammatory cytokine, another pro-inflammatory agent, MAM and Poly (I:C), under conditions suitable for influencing neuronal development, wherein the neuronal network activity is characterized by an excitatory and inhibitory imbalance. Alternatively, the neuronal network may be obtained from a subject suspected of having a condition associated with an E/I imbalance, or having such a condition.

The invention is further illustrated by the following examples. These are not intended to limit the scope of the invention. All references cited herein are herewith fully incorporated.

### 5 Examples

Following examples describe in vitro experiments with microelectrode arrays.

### 5.1 Primary cell cultures on MEA plates treated with a disease mimicking agent

Sterile MEA 48-well plates (Axion Biosciences) were first coated with polyethylimin (50% in borate buffer) to enhance cell adherence. The wells were washed three times with ultrapure water and then air-dried.

Cell material was prepared from NMRI mice (Charles River). Neuronal postmitotic cells were dissected at day 14 of the embryonic stage for combined midbrain and frontal cortex cultures or on embryonic days 16 for frontal cortex cultures.

The tissue was dissociated mechanically by enzymatically (133 Kunitz units/ml DNase; 10 units/ml papain) and mechanically by trituration, centrifuged and taken up in Dulbecco's Modified Eagle's Medium, DMEM, comprising laminin (10 µg/ml), 10% fetal calf serum and 10% horse serum. The cells in suspension were counted and tested for vitality and the concentration adjusted to 1 million cells/ml. One 20 µl drop was used per electrode array, i.e. 150.000 cells were cultured in each MEA well. After 1 hour for adherence of cells, medium was added ad 300 µl. The cells were incubated at 37°C at 10% CO₂. Half of the medium was exchanged twice a week with DMEM comprising 10% horse serum.

### 5.2 Disease model compounds

Polyclonal antibody to *Neisseria gonorrhoeae* (anti-NG, a-NG), was purchased from antibodies online, https://www.antibodies-online.com, order number: ABIN285584. ABIN285584 contains toxic sodium azide, which was extracted by centrifugation, as described (Reuss 2014).

The a-NG inactive was inactivated by boiling a-NG as a control. A second antibody, a-Actinin was purchased from Santa Cruz (order number sc-7210) and used as a second control. Phosphate-buffered saline, PBS, was purchased from Sigma-Aldrich and was used as a third control.

### 5.3 Culture treatment and recording

Cultures were treated with a-NG, a-NG inactive and a-Actin after 7 days in vitro with the medium change at a concentration of 10 µg / ml.

Recordings were performed with Maestro devices from Axion Biosystem, Atlanta, at day 11, 13-15, 18, 21 and 28 days in vitro for 60 minutes at minimum.

Spike train parameters were calculated from these recordings, s. **Fig. 1** and **Fig. 2****.** Based on this, an effect score was calculated as follows: The 20 most significant parameters were selected, and every parameter was scaled and shifted so that the mean of the control is 0.0, and the mean of a-NG is 1.0. For every data point, the 20 parameter values are scaled and averaged, yielding the effect scores. Effect score values for all data groups are averaged and analyzed statistically.

### 5.3.1 Assay optimization

The first aim of assay optimization was distinction of the normal (healthy/non-treated) phenotype and a phenotype which can be used as a disease model e.g., for schizophrenia (disease phenotype). Further, the assay was optimized to lead to relevant changes of the disease phenotype with a trend towards the normal phenotype.

The timing of the contact with the disease mimicking agent after start of cultivation of the neuronal progenitor cells can be decisive for affecting different developmental processes, and it can affect these to a different grade. Thus, contact between 4 and 10 days after start of the *in vitro* cultivation was tested.

The time of analysis was also optimized between 14 to 28 days after start of cultivation *in vitro* to find the period at which the changes due to the disease mimicking agent can be best determined. For each combination of time of contact and time of contact, the statistical separation of the two phenotypes is determined via a calculation of the Z' score, ***s. (Kummel, Gubler et al. 2010).***

Furthermore, the effect of the reaction to reference agents, e.g., known antipsychotic agents or relevant research substances which are described as affecting agonistic, antagonistic or modulatory receptors described as relevant for schizophrenia, is quantified.

Good results were, e.g., found when after 4 days in culture, the cells of some of the neuronal cell cultures were treated with a polyclonal antibody to *Neisseria gonorrhoeae,* as described above. After 28 days in cell culture, neuronal networks derived from cells contacted with the antibody to *Neisseria gonorrhoeae* show a distinctly different electrophysiological activity compared to neuronal networks not contacted with said antibody.

Interestingly, these activity patterns are similar to those patterns of activity in which the neuronal frontal cell cultures were treated with an NMDA antagonist. It could be demonstrated that these cultures also exhibit a disturbed E/I balance, with an excitatory predominance as in patients with schizophrenia.

### 5.4 Reference compounds

Neuronal cultures were cultivated for 28 days to be used for compound testing. Recordings were performed with Maestro devices from Axion Biosystem.

12 compounds comprising the two reference groups - excitatory and inhibitory - were applied cumulatively at concentrations spanning the entire response range (Table 2). For each concentration, one hour was recorded, and the last 30 min were analyzed.

**Table 2: Exemplary compounds**

| **Excitatory** | **Inhibitory** |
|---|---|
| AMPA | Bicuculline |
| Ketamine | Clonazepam |
| MK-801 | Diazepam |
| NBQX | Pentylenetetrazole |
| NMDA | Picrotoxin |
| | SKF 97541 |
| | Zolpidem |

**Table 3: Two and four class classifier for the E/I balance.**

| E/I Balance Distorsion Type | Type of Mechanism | Compounds |
|---|---|---|
| E - Excitatory | AT-I | PTZ - Pentylenetetrazole |
| | | PTX - Picrotoxin |
| | | Bicuculline |
| | AG-E | NMDA |
| | | AMPA |
| I - Inhibitory | AT-E | Ketamine |
| | | MK801 |
| | | NBQX |
| | AG-I | Diazepam |
| | | Clonazepam |
| | | Zolpidem |
| | | SKF97541 |

Where AT-I means caused by antagonism of inhibition, AG-E means agonism of excitation, AT-E means antagonism of excitation and AG-I means agonism of inhibition.

### 5.5 Machine learning for classification

Machine learning was used to train an artificial neural network with the reference data to recognize the two or four groups of Table 2 and Table 3. This artificial neural network was then used to classify the test data of the 4 antibody groups, i.e. assign their data to one of the two groups.

### 5.6 Classification results of E/I balance classifier

### 5.6.1 Example 1 - Frontal cortex

A significant effect was observed at day 28 in vitro, cf. **Fig. 5****.** The antibody treatment with a-NG induced a significantly different activity pattern compared to PBS, a-NG inactive and a-Actin. In conclusion, days 24-32, in particular, day 28 in vitro was selected as a preferred read out day.

Spike train parameters at DIV 28 were computed. Burst parameters, which are correlated with theta oscillation (Lisman, Coyle et al.) were obviously increased as Burst parameter: Burst rate, Burst Spike rate and Burst Spike Density for a-NG treated cultures in contrast to their controls. This is a first clear indication, that these cultures show an electro-physiological phenotype as were described for schizophrenic like behaviour (Lisman, Coyle et al.) (Uhlhaas).

A second indication could be delivered by an E/I balance analysis.

We classified the 28 days in vitro data with our E/I Balance classifier. Results are shown in Table 5. First, an E/I balance classifier was established with data sets of cultures treated as listed in Table 2. For this, the accuracy of this method was tested in a cross validation approach of 10 % of data leave out. In Table 4 it is shown that the accuracy is with more than 80% high enough.

**Table 4: Cross Validation results**

| | **Class E** | **Class I** | **data points** |
|---|---|---|---|
| **Class E** | 82% | 18% | 334 |
| **Class I** | 14% | 86% | 400 |
| **data points** | 333 | 401 | 734 |

After cross validation, a classifier was finally trained with all data sets from the learning data set. With this classifier, all data records treated with the four groups as in Table 2 were classified.

**Table 5: Classification results**

| **antibody group** | **data points** | **Class E** | **Class I** |
|---|---|---|---|
| **anti-NG, active** | **11** | **5** = 45% | **6** = 55% |
| **anti-NG, inactivated** | **11** | **3** = 27% | **8** = 73% |
| **control PBS** | **11** | **3** = 27% | **8** = 73% |
| **anti-Actinin** | **11** | **3** = 27% | **8** = 73% |

Data sets from cultures treated with Neisseria antibody in its active form, ant-NG, active, showed an increased excitatory behavior, Class E, of 45% of all data records than the control data sets with inactived anti-NG, only PBS treated cultures or with another antibody anti-Actinin. This demonstrates that the E/I balance was shifted to the excitatory side.

### 6 Literature

Almamy, A., C. Schwerk, H. Schroten, H. Ishikawa, A. R. Asif and B. Reuss (2017). "Crossreactivity of an Antiserum Directed to the Gram-Negative Bacterium Neisseria gonorrhoeae with the SNARE-Complex Protein Snap23 Correlates to Impaired Exocytosis in SH-SY5Y Cells." J Mol Neurosci 62(2): 163-180.
Babulas, V., P. Factor-Litvak, R. Goetz, C. A. Schaefer and A. S. Brown (2006). "Prenatal Exposure to Maternal Genital and Reproductive Infections and Adult Schizophrenia." American Journal of Psychiatry 163(5): 927-929.
Bader, B. M., A. Steder, A. B. Klein, B. Frolund, O. H. U. Schroeder and A. A. Jensen (2017). "Functional characterization of GABAA receptor-mediated modulation of cortical neuron network activity in microelectrode array recordings." PLoS One 12(10): e0186147.
Brennand, K. J., A. Simone, J. Jou, C. Gelboin-Burkhart, N. Tran, S. Sangar, Y. Li, Y. Mu, G. Chen, D. Yu, S. McCarthy, J. Sebat and F. H. Gage (2011). "Modelling schizophrenia using human induced pluripotent stem cells." Nature 473(7346): 221-225.
Feng, X., B. M. Bader, F. Yang, M. Segura, L. Schultz, O. H. U. Schröder, A. Rolfs and J. Luo (2018). "Improvement of impaired electrical activity in NPC1 mutant cortical neurons upon DHPG stimulation detected by micro-electrode array." Brain Research 1694: 87-93.
Ford, T. C., R. Nibbs and D. P. Crewther (2017). "Increased glutamate/GABA+ ratio in a shared autistic and schizotypal trait phenotype termed Social Disorganisation." Neuroimage Clin 16: 125-131.
Frohlich, J. and J. D. Van Horn (2014). "Reviewing the ketamine model for schizophrenia." J Psychopharmacol 28(4): 287-302.
Gao, R. and P. Penzes (2015). "Common Mechanisms of Excitatory and Inhibitory Imbalance in Schizophrenia and Autism Spectrum Disorders." Current molecular medicine 15(2): 146-167. Gogolla, N., J. J. Leblanc, K. B. Quast, T. C. Sudhof, M. Fagiolini and T. K. Hensch (2009). "Common circuit defect of excitatory-inhibitory balance in mouse models of autism." J Neurodev Disord 1(2): 172-181.
Hölscher, C., R. Anwyl and M. J. Rowan (The Journal of Neuroscience). "Stimulation on the Positive Phase of Hippocampal Theta Rhythm Induces Long-Term Potentiation That Can Be Depotentiated by Stimulation on the Negative Phase in Area CA1 In Vivo." The Journal of Neuroscience: 6470-6477.
Jacobs, B. M. (2015). "A dangerous method? The use of induced pluripotent stem cells as a model for schizophrenia." Schizophr Res 168(1-2): 563-568.
Jones, C., D. Watson and K. Fone (2011). "Animal Models of Schizophrenia." British Journal of Pharmacology 164(4).
Kehrer, C., N. Maziashvili, T. Dugladze and T. Gloveli (2008). "Altered Excitatory-Inhibitory Balance in the NMDA-Hypofunction Model of Schizophrenia." Front Mol Neurosci 1: 6.
Kummel, A., H. Gubler, P. Gehin, M. Beibel, D. Gabriel and C. N. Parker (2010). "Integration of multiple readouts into the z' factor for assay quality assessment." J Biomol Screen 15(1): 95-101.
Lisman, J. E., J. T. Coyle, R. W. Green, D. C. Javitt, F. M. Benes, S. Heckers and A. A. Grace (2008). "Circuit-based framework for understanding neurotransmitter and risk gene interactions in schizophrenia." Trends in Neurosciences 31(5): 234-242.
Parenti, C., R. Turnaturi, G. Arico, A. Gramowski-Voss, O. H. Schroeder, A. Marrazzo, O. Prezzavento, S. Ronsisvalle, G. M. Scoto, G. Ronsisvalle and L. Pasquinucci (2013). "The multitarget opioid ligand LP1's effects in persistent pain and in primary cell neuronal cultures." Neuropharmacology 71: 70-82.
Piontkewitz, Y., M. Arad and I. Weiner (2012). "Tracing the development of psychosis and its prevention: What can be learned from animal models." Neuropharmacology 62(3): 1273-1289. Remington, G. (2015). "Extrapyramidal Side Effects." Canadian Journal of Psychiatry. Revue Canadienne de Psychiatrie 60(9): 412-412.
Reuss, B. (2014). "Antibodies directed to Neisseria gonorrhoeae impair nerve growth factor-dependent neurite outgrowth in Rat PC12 cells." J Mol Neurosci 52(3): 353-365.
Reuss, B., A. R. Asif, A. Almamy, C. Schwerk, H. Schroten, H. Ishikawa, C. Drummer and R. Behr (2016). "Antisera against Neisseria gonorrhoeae cross-react with specific brain proteins of the common marmoset monkey and other nonhuman primate species." Brain Res 1653: 23-38.
Shi, Y., H. Inoue, J. C. Wu and S. Yamanaka (2017). "Induced pluripotent stem cell technology: a decade of progress." Nat Rev Drug Discov 16(2): 115-130.
Swinney, D. C. (2013). "The contribution of mechanistic understanding to phenotypic screening for first-in-class medicines." J Biomol Screen 18(10): 1186-1192.
Turrigiano, G. G., K. R. Leslie, N. S. Desai, L. C. Rutherford and S. B. Nelson (1998). "Activity-dependent scaling of quantal amplitude in neocortical neurons." Nature 391(6670): 892-896. Uhlhaas, P. J. (2013). "Dysconnectivity, large-scale networks and neuronal dynamics in schizophrenia." Curr Opin Neurobiol 23(2): 283-290.
Weinert, M., T. Selvakumar, T. S. Tierney and K. N. Alavian (2015). "Isolation, culture and long-term maintenance of primary mesencephalic dopaminergic neurons from embryonic rodent brains." Journal of visualized experiments : JoVE(96): 52475.
US 2018/0149639 A1
US 2017/0326356 A1
WO99/20315 A1
WO 01/56647 A1
WO 2016/028880 A1

## Claims

1. A neuronal network comprising excitatory and inhibitory neurons *in vitro,* wherein the neuronal network is derived from cells contacted with a disease mimicking agent selected from the group consisting of antibodies to *Neisseria gonorrhoeae,* antibodies to *Herpes simplex,* LPS, a pro-inflammatory cytokine and another pro-inflammatory agent, MAM and Poly (I:C), under conditions suitable for influencing neuronal development, wherein the neuronal network activity is **characterized by** an excitatory and inhibitory imbalance.

2. A method for analyzing excitatory and inhibitory balance in neuronal networks comprising excitatory and inhibitory neurons, wherein, optionally, the method is an *in vitro* method and the neuronal networks are in cell culture, the method comprising
a) detecting an electrophysiological activity of a neuronal network under conditions of
i) stimulation of the excitatory neurons with a first stimulus; wherein a first activity is detected; and/or
ii) inhibition of the excitatory neurons with a second stimulus; wherein a second activity is detected; and/or
iii) stimulation of the inhibitory neurons with a third stimulus; wherein a third activity is detected; and/or
iv) inhibition of the inhibitory neurons with a fourth stimulus; wherein a fourth activity is detected;
wherein the electrophysiological activity is at least detected under conditions of i) or iv) and under conditions of ii or iii), wherein, preferably, the electrophysiological activity is detected under conditions i), ii), iii) and iv).

3. A method for analyzing excitatory and inhibitory balance in a neuronal network of interest comprising excitatory and inhibitory neurons, wherein, optionally, the method is an *in vitro* method and the neuronal network is in cell culture, said method comprising
a) detecting a basic electrophysiological activity of said neuronal network;
b) carrying out the method of claim 2 with neuronal networks derived from at least one reference subject, preferably, a healthy reference subject;
wherein steps a) and b) can be carried out in any order;
c) comparing said basic electrophysiological activity of step a) with the electrophysiological activities determined in step b).

4. A method for analyzing excitatory and inhibitory balance in a neuronal network of interest comprising excitatory and inhibitory neurons, wherein, optionally, the method is an *in vitro* method and the neuronal network is in cell culture, the method comprising
a) detecting a basic electrophysiological activity of said neuronal network;
b) comparing said basic electrophysiological activity with the electrophysiological activities determined by carrying out the method of claim 2 with neuronal networks derived from at least one reference subject, preferably, a healthy reference subject.

5. The method of any of claims 3 or 4, further comprising contacting the neuronal network of interest or a neuronal network having the same origin as the neuronal network of interest with a potential antipsychotic agent and detecting the electrophysiological activity of said network under the influence of said potential antipsychotic agent.

6. The method of any of claims 2-5, wherein the first, second, third and/or fourth stimulus is a chemical or biological agent selected from the group comprising a neurotransmitter, an inhibitor of a neurotransmitter, an antibody, a RNA interference approach with shRNA, and CRISPR/CAS,
wherein the first stimulus is a GABA receptor antagonist selected from the group comprising Pentylentetrazole, Picrotoxin and Bicuculline, and/or
wherein the second stimulus is a Glutamate receptor agonist selected from the group comprising NMDA (N-Methyl-D-aspartic acid) and AMPA (α-amino-3-hydroxy-5-methyl-4-isoxazolepropionic acid), , and/or
wherein the third stimulus is a NMDA or AMPA receptor antagonist selected from the group comprising Ketamine, MK801 or NBQX, and/or
wherein the fourth stimulus is a GABA-A or GABA-B receptor agonist selected from the group comprising Diazepam, Clonazepam, Zolpidem or SKF97541.

7. The method of any of claims 2-6, wherein the electrophysiological activity is determined by a method selected from the group consisting of spike train activity, calcium imaging and field potential recording, preferably, by spike train analysis.

8. The method of any of claims 2-7, wherein said comparing step is carried out with a computer program product suitable for comparing spike train patterns selected from the group of computer program products comprising a spike train learner and a spike train model, wherein the computer program product preferably uses artificial intelligence trained on reference neuronal networks for said comparison.

9. The method of any of claims 2-8, wherein the neuronal network is derived from cells contacted with a disease mimicking agent selected from the group consisting of antibodies to *Neisseria gonorrhoeae,* antibodies to *Herpes simplex* or LPS, a pro-inflammatory cytokine and another pro-inflammatory agent, MAM and Poly (I:C), under conditions suitable for influencing neuronal development, wherein, preferably, the neuronal network is derived from cells contacted with said agent *in vitro.*

10. The neuronal network of claim 1 or the method of any of claims 2-9, wherein the neuronal network is derived from cells contacted with antibodies to *Neisseria gonorrhoeae* selected from the group comprising polyclonal antibodies, wherein, preferably, the neuronal network is derived from cells contacted with said antibodies *in vitro.*

11. The neuronal network of any of claims 1 or 10 or the method of any of claims 2-10, wherein the neuronal network is derived from cells of a subject suspected of having a condition or having a condition associated with an excitatory and inhibitory imbalance selected from the group comprising schizophrenia, an autism spectrum disorder, bipolar disorder, Alzheimer's disease, Parkinson's disease, ADS, ADHS and depression.

12. The neuronal network of any of claims 1 or 10-11 or the method of any of claims 2-11, wherein the neuronal network is derived from cells of a subject selected from the group consisting of neuronal progenitor cells, PSC, preferably, iPSC, and non-human embryonic stem cells.

13. The neuronal network of any of claims 1 or 10-12 or the method of any of claims 2-12, wherein the neuronal network comprises cells derived from a human, a mouse, a rat, a rabbit, a guinea pig, a dog, a cat, a monkey, an ape or a pig, preferably, a human.

14. The neuronal network of any of claims 1 or 10-13 or the method of any of claims 2-13, wherein the neuronal network comprises glutaminergic neurons, GABAergic neurons and, preferably, dopaminergic neurons.

15. The neuronal network of any of claims 1 or 10-14 or the method of any of claims 2-14, wherein the neuronal network is in contact with a multi-electrode array.

16. A method of identifying an antipsychotic agent, comprising carrying out the method of any of claims 5 to 15.

17. A method of selecting a drug suitable for treatment of a subject having a condition associated with an excitatory and inhibitory imbalance in a subject, comprising
carrying out the method of any of claims 5-15 with a neuronal network of interest comprising excitatory and inhibitory neurons from cells derived from cells of the subject, preferably, from iPSC,
wherein the method comprises contacting said neuronal network with at least one drug, preferably, an antipsychotic agent, and detecting the electrophysiological activity of said network under the influence of said drug,
wherein a drug is suitable for treatment of the condition if the excitatory and inhibitory imbalance in the neuronal network is ameliorated by said drug.

18. An *in vitro* method for diagnosing a condition associated with an excitatory and inhibitory imbalance in a subject suspected of having a condition associated with an excitatory and inhibitory imbalance selected from the group comprising schizophrenia, autism, bipolar disorder, Alzheimer's disease, Parkinson's disease, ADS, ADHS and depression, comprising
A) optionally, generating a neuronal network of interest comprising excitatory and inhibitory neurons from cells derived from cells of the subject, preferably, from iPSC,
B) carrying out the method of any of claims 11-15, with the neuronal network of interest derived from cells of the subject in step A), wherein an excitatory and inhibitory imbalance detected in said neuronal network is associated with said condition,
C) optionally, classifying the excitatory and inhibitory imbalance detected in said neuronal network is according to a comparison with the results of an analysis of at least one reference subject having said condition, wherein, preferably, said comparison is carried out by a machine learning algorithm or pattern recognition algorithm,
D) optionally, further providing the diagnosis to the subject or a medical practitioner responsible for the subject.

19. A stimulus suitable for
i) stimulation of excitatory neurons, designated the first stimulus; and/or
ii) inhibition of excitatory neurons, designated the second stimulus; and/or
iii) stimulation of the inhibitory neurons, designated the third stimulus; and/or
iv) inhibition of the inhibitory neurons, designated the fourth stimulus;
wherein the first, second, third and fourth stimulus is a chemical or biological agent selected from the group comprising a neurotransmitter, an inhibitor of a neurotransmitter, an antibody, an RNA interference approach, and CRISPR/CAS,
for use in diagnosing a condition associated with an excitatory and inhibitory imbalance, by analyzing excitatory and inhibitory balance in a neuronal network of interest comprising excitatory and inhibitory neurons derived from cells of a subject suspected of having a condition associated with an excitatory and inhibitory imbalance selected from the group comprising schizophrenia, autism, bipolar disorder, Alzheimer's disease, Parkinson's disease, ADS, ADHS and depression, comprising
a) detecting a basic electrophysiological activity of said neuronal network of interest *in vivo* using a multi-electrode array in contact with said neuronal network;
b) detecting an electrophysiological activity of reference neuronal networks under conditions of
i) stimulation of the excitatory neurons with the first stimulus; wherein a first activity is detected; and/or
ii) inhibition of the excitatory neurons with the second stimulus; wherein a second activity is detected; and/or
iii) stimulation of the inhibitory neurons with the third stimulus; wherein a third activity is detected; and/or
iv) inhibition of the inhibitory neurons with the fourth stimulus; wherein a fourth activity is detected;
wherein the electrophysiological activity is at least detected under conditions of i) or iv) and under conditions of ii or iii), wherein, preferably, the electrophysiological activity is detected under conditions i), ii), iii) and iv)
wherein steps a) and b) can be carried out in any order;
c) comparing said basic electrophysiological activity of step a) with the electrophysiological activities determined in step b).
d) optionally, classifying the excitatory and inhibitory imbalance detected in said neuronal network of interest according to a comparison with the results of an analysis of at least one reference subject having said condition, wherein, preferably, said comparison is carried out by a machine learning or pattern recognition algorithm,
e) optionally, further providing the diagnosis to the subject or a medical practitioner responsible for the subject.

20. Use of the neuronal network of any of claims 1 or 10-14 for analyzing excitatory and inhibitory balance in a neuronal network, preferably, for identifying an antipsychotic agent.

21. Use of a the method of any of claims 2-15 for analyzing excitatory and inhibitory balance in a neuronal network, preferably, for identifying an antipsychotic agent, wherein, optionally, the neuronal network is the neuronal network of any of claims 1 or 10-14.
